(19) Europäisches Patentamt
European Patent Office
Office européen des brevets

(11) Veröffentlichungsnummer: **0 542 145 A1**

(12) # EUROPÄISCHE PATENTANMELDUNG

(21) Anmeldenummer: **92119025.2**

(22) Anmeldetag: **06.11.92**

(51) Int. Cl.⁵: **C07H 5/06**, C07H 1/08, C07K 15/14, C07K 15/16, C07K 15/28, A61K 39/385, G01N 33/541

(30) Priorität: **11.11.91 DE 4137236**

(43) Veröffentlichungstag der Anmeldung: **19.05.93 Patentblatt 93/20**

(84) Benannte Vertragsstaaten: **AT BE CH DE DK ES FR GB GR IE IT LI LU NL PT SE**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Strecker, Gérard, Dr.**
**4, Rue de la Carcasse**
**F-59235 Bersée(FR)**
Erfinder: **Michalski, Jean-Claude, Dr.**
**27, Rue de la Porte d'Ypres**
**F-5900 Lille(FR)**
Erfinder: **Montreuil, Jean, Prof. Dr.**
**145, Rue Jules Boucly**
**F-59650 Villeneuve(FR)**
Erfinder: **Kordowicz, Maria, Dr.**
**In den Wingerten 31**
**W-6100 Darmstadt(DE)**

(54) **Oligosaccharide mit antigenen Determinaten aus der Schleimhaut von Amphibieneiern.**

(57) Die Erfindung betrifft neue fucosylierte Oligosaccharide, die in beträchtlichen Mengen aus der Schleimhülle von Eigelegen von Amphibien, vorzugsweise der Familie der Molche, isoliert werden können und deren Strukturen antigene Determinanten aufweisen und somit für die Herstellung von mono- und polyklonalen, insbesondere anti-$Le^X$-, anti-$Le^Y$- und anti-KDN-Antikörpern, sowie für die Bereitstellung entsprechender Immunokonjugate, Immunoadsorbentien, Immunoassays und Impfstoffe für die Tumortherapie geeignet sind.

EP 0 542 145 A1

Die Erfindung betrifft neue antigene Determinanten enthaltende Oligosaccharide im wesentlichen in reiner Form der folgenden Formeln:

(A)

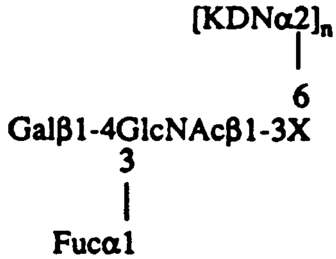

(B)

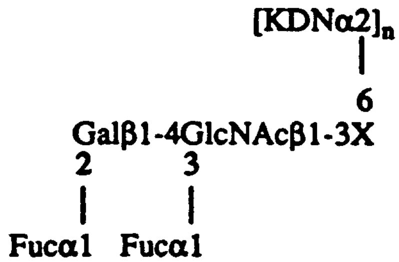

(C)

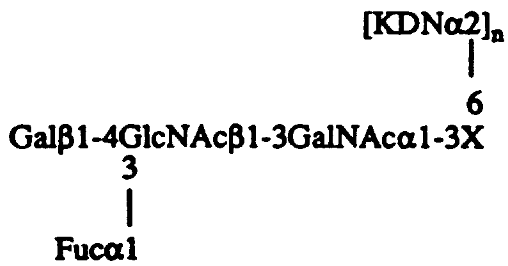

(D)

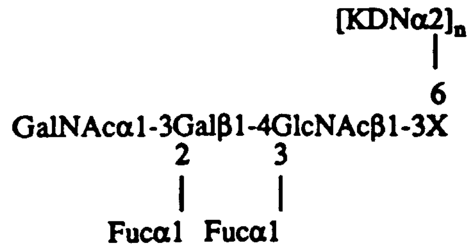

(E)

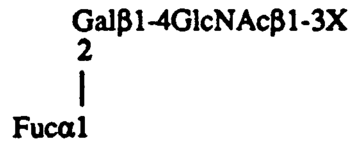

(F)

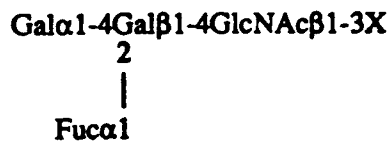

(G)

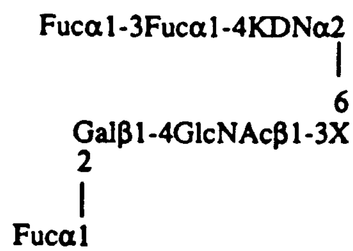

(H)

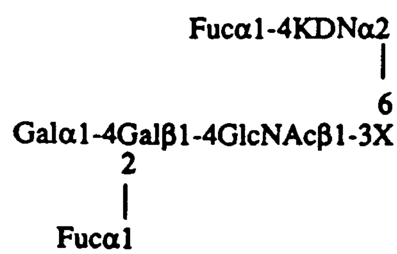

(I)

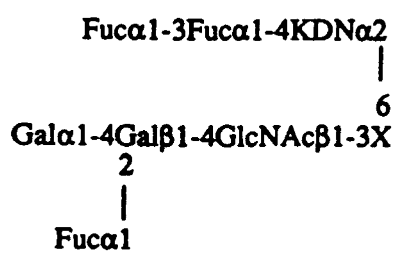

worin

| | |
|---|---|
| X | GalNAc oder GalNAc – ol, |
| n | 0 oder 1, |
| Gal | D – Galactose, |
| GlcNAc | N – Acetyl – D – glucosamin, |
| GalNAc | N – Acetyl – D – galactosamin, |

GalNAc-ol N-Acetyl-D-galactosaminitol,
Fuc L-Fucose und
KDN 3-Deoxy-D-glycero-D-galacto-nonulosonsäure
bedeuten.

Die Erfindung betrifft ferner ein Verfahren zur Herstellung der Oligosaccharide (A) bis (I), das dadurch gekennzeichnet ist, daß man die Schleimhülle von Eigelegen von Amphibien, insbesondere der Familie der Molche, isoliert und die in ihr enthaltenen Glycoproteine (Mucine) chemisch, insbesondere durch reduktive $\beta$-Eliminierung, spaltet und das Gemisch der entstandenen freien Oligosaccharide in nativer Form bzw. in Form der Alditole chromatographisch aufreinigt und gegebenenfalls im Falle der Verbindungen der Formeln (A) bis (D), worin n = 0 bedeutet, den KDN-Rest chemisch abspaltet.

Die Erfindung betrifft außerdem Neoglycokonjugate, enthaltend eine Zucker-Komponente und einen Träger, insbesondere ein Protein oder Peptid, wobei gegebenfalls die Zucker-Komponente über einen Linker an den Träger gekoppelt ist und ein Oligosaccharid der Formeln (A) bis (I) ist.

Ferner betrifft die Erfindung einen Impfstoff, enthaltend im wesentlichen ein oben bezeichnetes Neoglycokonjugat.

Die Erfindung betrifft auch einen mono- oder polyklonalen Antikörper, der dadurch gekennzeichnet ist, daß er spezifisch auf eine antigene Determinante eines Oligosaccharides der Formeln (A) bis (I) gerichtet ist.

Die Erfindung betrifft weiterhin tumorassoziierte Antigene, die ein Epitop enthalten, an das ein oben bezeichneter mono- oder polyklonaler Antikörper bindet.

Überdies betrifft die Erfindung Immunadsorbentien zur Aufreinigung der oben bezeichneten Antikörper, welche aus einer Trägermatrix bestehen, an die die Oligosaccharide der Formeln (A) bis (I) gekoppelt sind, sowie ferner Immunadsorbentien zur Aufreinigung der Oligosaccharide der Formeln (A) bis (I), welche aus einer Trägermatrix bestehen, an die die oben genannten Antikörper gekoppelt sind.

Die Erfindung betrifft ferner ein Verfahren zur in vitro-Bestimmung von Immunogenität durch antigen-spezifische Stimulation von isolierten Lymphocyten, das dadurch gekennzeichnet ist, daß man die Lymphocyten mit dem Mucin aus der Schleimhülle von Amphibien-Eiern oder mit einem oben bezeichneten Neoglycokonjugat rekultiviert und die Proliferation der Lymphocyten durch Aufnahme von $[^3H]$-Thymidin mißt.

Schließlich betrifft die Erfindung diverse neue Verwendungen der Schleimhülle von Eigelegen von Amphibien, insbesondere aus der Familie der Molche, so deren Verwendung

- zur Gewinnung von Oligosacchariden mit dem Strukturelement Gal($\beta$1-4) - GlcNAc($\beta$1-3), wobei mindestens zusätzlich ein Fuc-Rest und mindestens ein weiterer Gal-, GalNAc-, GlcNAc-, Fuc- oder KDN- Rest mit den oben angegebenen Bedeutungen vorliegt;
- zur Herstellung von anti-$Le^X$-, anti-$Le^Y$- und anti-KDN-Antikörpern;
- zur Herstellung von 3-Desoxy-D-glycero-D-galacto-nonulosonsäure (KDN);
- zur Bereitstellung eines Immunoassays und
- zur Herstellung eines Impfstoffes für die Behandlung von Tumoren.

Glycoproteine und Glycolipide (Glycokonjugate) sind in der Natur weit verbreitet. Sie spielen in der Biochemie der lebenden Materie die verschiedenartigsten Rollen, wobei ihre große Bedeutung nicht zuletzt dem unterschiedlichen Charakter von polarer Kohlenhydrat-Hälfte (Glycan) und unpolarer Protein- bzw. Lipid-Hälfte in einem Molekül zuzuschreiben ist.

So ist es bekannt, daß Strukturen im Glycan-Teil Epitope darstellen, die identisch sind mit den antigenen Determinanten, die zuweilen auf Gewebeoberflächen, insbesondere auf Tumorzellen (tumor-assozierte Antigene) zu finden sind. Dies bedeutet, daß auf Tumorzellenoberflächen gewöhnlich eine bedeutend größerer Anzahl von bestimmten Glycoproteinen oder Glycolipiden mit antigener Wirkung zu beobachten sind als auf gesunden Zellen.

Im Falle der tumor-assoziierten Antigene konnte festgestellt werden, daß sie im allgemeinen als entwicklungsregulierte Antigene exprimiert und daher auch als oncofetale Antigene bezeichnet werden (Hakomori, 1989, Adv. Cancer Res. 52, 257).

Einige Strukturen oder Teilstrukturen im Glycan-Teil sind bereits als antigene Determinanten näher charakterisiert worden. So ist die Lewis X ($Le^X$ bzw. SSEA-1) Determinante auf der Oberfläche von zahlreichen sich differenzierenden Zellen nachgewiesen worden (z. B. Feizi et al., 1981, Nature 292, 5819; Hakomori, 1984, J. Biol. Chem. 259, 4672; Fukushi et al., 1984, J. Biol. Chem. 259, 4681). Eine andere, als Lewis Y ($Le^Y$) bezeichnete Determinante sowie eine als A-$Le^Y$ bezeichnete Variante davon sind auf verschiedenen Krebszellen lokalisiert und identifiziert worden (z.B. Hakomori, 1989, Adv. Cancer Res. 52, 257; Magnani et al., 1983, Cancer Res. 43, 5489; Abe et al., 1983, J. Biol. Chem. 258, 11793; Nudelman et al., 1986, J. Biol. Chem. 261, 11247).

Mit $Le^X$ wird das Strukturelement Gal(β1 – 4) – [Fuc(α1 – 3)] – GlcNAc(β1 – 3), mit $Le^Y$ das Strukturelement Fuc(α1 – 2) – Gal(β1 – 4) – [Fuc(α1 – 3)] – GlcNAc(β1 – 3) und mit A – $Le^Y$ das Strukturelement GalNAc – (α1 – 3) – [Fuc(α1 – 2)] – Gal(β1 – 4) – [Fuc(α1 – 3)] – GlcNAc(β1 – 3) mit den oben angegebenen Bedeutungen bezeichnet.

In vielen der untersuchten natürlich vorkommenden Glycokonjugaten mit antigener Wirkung ist der Glycan – Teil an eine Proteinmatrix (O – oder N – glycosidisch) gebunden. Als terminaler Zuckerrest tritt häufig GlcNAc – oder GalNAc – auf. Anordnungen wie GalNAc(α1 – 3) – GalNAc(α1 – 3) – Ser(Thr) wurden auch in humanen Darmkrebszellen gefunden.

Oligosaccharide bzw. Glycokonjugate mit antigenen Determinanten stellen also wichtige Verbindungen für die diagnostische Medizin, die klinische Pharmakologie sowie die Therapie von Krebserkrankungen dar. Es besteht somit naturgemäß ein Bedürfnis entsprechende Verbindungen in die Hand zu bekommen. Aus geeignetem Gewebe lassen sich solche Verbindungen nur umständlich und vor allem nur in geringen Mengen gewinnen. Überdies ist Tumorgewebe kaum zugänglich. Ein weitere Methode ist prinzipell in der chemischen Synthese der gewünschten Oligosaccharide zusehen. Derartige Synthesen weisen jedoch den Nachteil auf, daß sie über viele teilweise aufwendige Zwischenstufen laufen, wobei insbesondere auf die Stereochemie geachtet werden muß. Dies hat zur Folge, daß die Ausbeuten meist bescheiden sind.

Es bestand somit die Aufgabe, Oligosaccharide bzw. entsprechende Konjugate mit antigenen Determinanten auf einfache, preiswerte Art und in wirtschaftlich bedeutenden Mengen zur Verfügung zu stellen.

Es wurde nun gefunden, daß aus der Schleimhülle von Eigelegen von Amphibien, insbesondere aus der Familie der Molche, sehr einfach und in überraschend hohen Mengen und Ausbeuten Oligosaccharide durch chemische Abspaltung aus dem in der Schleimhülle befindlichen Mucin gewonnen werden können. Die erfindungsgemäßen Oligosaccharide weisen alle das Strukturelement Gal(β1 – 4) – GlcNAc(β1 – 3), sowie einen GalNAc oder GalNAc – ol Rest auf, über den die Bindung zum Proteinanteil des Mucins erfolgt, und sind zudem fucosyliert. Die Verbindungen setzen sich allgemein aus den Komponenten Gal, GalNAc (Gal – NAc – ol), GlcNAc, Fuc und teilweise zusätzlich aus KDN zusammen.

Im einzelnen wurden in den Molchgattungen Pleurodeles und Axolotl die Verbindungen der Formeln (A) bis (I) isoliert und charakterisiert. Es muß aber herausgestellt werden, daß erfindungsgemäß die Schleimhülle von Amphibieneiern generell eine reichhaltige Quelle von antigene Determinanten tragenden fucosylierten Oligosacchariden unterschiedlichster Strukturen, die sich aus den oben genannten Einzelkomponenten zusammensetzen, darstellt.

Viele der in der Schleimhülle von Amphibieneiern sich befindlichen Glycokonjugate enthalten im Glycan – Teil den KDN – Rest. KDN, d.h. 3 – Deoxy – D – glycero – D – galacto – nonulosonsäure, ist ein deaminiertes Derivat der natürlich vorkommenden Neuraminsäure. KDN konnte bislang nur im Laich von Forellen und Lachsen nachgewiesen und in geringen Mengen daraus isoliert werden (Nadano et al., 1986, J. Biol. Chem. 261, 11550; Kanamori et al., 1990, J. Biol. Chem. 265, 21811; Inoue et al., 1991, Proc. XIth Int. Symp. Glycoconj., Toronto). KDN wird als eine Verbindung angesehen, die bezüglich ihrer direkten oder indirekten antigenen Wirkung (Spezifität, Affinität) eine wichtige Rolle spielen soll. Synthetisch läßt sich KDN nur schwer herstellen. Daher ist erfindungsgemäß die Schleimhülle von Amphibieneiern auch eine willkommene Quelle für die Gewinnung von KDN.

Es wurde gefunden, daß der Kohlenhydratanteil der Schleimhülle von Amphibieneiern zwischen 45 und 55 %, insbesondere zwischen 48 und 52 % beträgt, unabhängig davon, um welche Amphibienart es sich handelt. Da die Schleimhülle selbst einen großen Anteil am Eigelege ausmacht, bedeutet dies, daß große Mengen von Glycan erhalten werden können. So können beispielsweise aus 160 mg (Trockengewicht) der Schleimhülle von Pleurodeles waltlii zwischen 16 und 25 mg der reinen Verbindungen der Formeln (A) bis (D) erhalten werden, wobei von jeder der erfindungsgemäßen Verbindungen zwischen 4 und 6 mg isoliert werden können. Der Anteil der vier Verbindungen (A) bis (D) an der Gesamtmenge Glycan beträgt also 10 bis 15 %.

Die Verhältnisse bei Axolotl mexicanum sind im Prinzip vergleichbar, wenn auch der Anteil der Verbindungen (E) bis (I) am Gesamtglycan hier niedriger ist, da nur ein Teil der aufgetrennten Oligosaccharide charakterisiert bzw. identifiziert wurde.

Es wurde ferner gefunden, daß die erfindungsgemäßen Oligosaccharide der Formeln (A) bis (D) geeignet sind, um Neokonjugate, wie Neoglycopeptide, Neoglycoproteine oder Neoglycolipide herzustellen. Da die Oligosaccharide als solche lediglich als Hapten fungieren, also keine Immunantwort auslösen, ist es notwendig, die erfindungsgemäßen Glycanreste direkt oder gegebenenfalls über Linker an geeignete Proteine, Peptide oder Lipide zu koppeln. Die so hergestellten neuen Neokonjugate können wiederum zur Herstellung eines Impfstoffes, der ein oder mehrere dieser Neokonjugate enthält, sowie zur Herstellung von mono – oder polyklonalen Antikörpern, die mit den antigenen Determinanten des Glycanteils dieser Konjugate in Wechselwirkung treten können, eingesetzt werden.

Weiter wurde gefunden, daß entsprechende Immunadsorbentien, die zur Reinigung entsprechender Proben geeignet sind, auf einfache Weise hergestellt werden können.

Für die Aufreinigung von Antikörperpräparationen wird ein erfindungsgemäßes Oligosaccharid an eine Trägermatrix gebunden; geeignete Trägermaterialien, Kopplungsverfahren und Reinigungsverfahren sind dem Fachmann bekannt. Für die Aufreinigung von Oligosacchariden oder Neoglykokonjugaten wird ein Antikörper an eine Trägermatrix gebunden; dazu geeignete Trägermaterialien, Kopplungsverfahren und Reinigungsverfahren sind dem Fachmann ebenfalls bekannt.

Schließlich wurde gefunden, daß humane Lymphocyten in vitro durch die erfindungsgemäßen Neoglycokonjugate oder die entsprechenden nativen Glycokonjugate (Mucin) stimuliert werden können, was durch den Einbau von markiertem Thymidin quantitativ gemessen werden kann.

Im folgenden werden die in der Anmeldung verwendeten Abbildungen erläutert:

FIG. 1: HPLC-Diagramm des Oligosaccharid-Alditol-Gemisches aus Pleurodeles waltlii. Letzteres wird durch reduktive $\beta$-Eliminierung aus dem Lyophilisat der Schleimhülle von Eiern von Pleurodeles waltlii erhalten. Als Träger dient ein Kieselgel mit einer Aminopropylsilyl-Phase (SUPELCOSIL$^R$ LC-$NH_2$, Fa. Supelco, Schweiz); Laufmittel: $CH_3CN$ / $KH_2PO_4$ (30 mM, pH 7.0) = 65 : 35 (v/v); 1 ml/min; Detektion: 206 nm.
Peak I: Verbindung der Formel (A);
Peak II: Verbindung der Formel (B);
Peak III: Verbindung der Formel (C);
Peak IV: Verbindung der Formel (D).

FIG. 2 : GC-Diagramm des derivatisierten Oligosaccharid-Alditol-Gemisches (Trifluoracetate der Methylglycoside) aus Pleurodeles waltlii. Das Oligosaccharid-Gemisch wird durch Methanolyse aus dem Lyophilisat der Schleimhülle von Eiern von Pleurodeles waltlii erhalten. Die Probenvorbereitung erfolgt nach Zanetta et al., 1972 (J. Chromatogr. 69, 291). Säule: Chromosorb Q, Silicon 5 %, OV 101 (1 m x 0.32 cm), Varian GC.
Als Standard dient Mesoinositol. Fuc, Gal, GlcNAc, GalNAc und KDN haben die oben angegebenen Bedeutungen.

FIG. 3 : HPLC-Diagramme des Oligosaccharid-Alditol-Gemisches aus Axolotl mexicanum. Letzteres wird durch reduktive $\beta$-Eliminierung aus dem Lyophilisat der Schleimhülle von Eiern von Axolotl mexicanum erhalten. Als Träger dient ein Kieselgel mit einer Aminopropylsilyl-Phase (SUPELCOSIL$^R$ LC-$NH_2$, Fa. Supelco, Schweiz);
(a) Diagramm des Gemisches der sauren Fraktion II (siehe Text, Beispiele).
Peak A3: Verbindung der Formel (G);
Peak A5: Verbindung der Formel (H);
Peak A6: Verbindung der Formel (I);
Peaks A1, A2, A4 wurden nicht weiter untersucht.
Laufmittel: $CH_3CN$ / $KH_2PO_4$ (30 mM, pH 7.0) = 65 : 35 (v/v); 1 ml/min; Detektion: UV 206 nm.
(b) Diagramm des Gemisches der neutralen Fraktion III (siehe Text, Beispiele).
Peak N3: Verbindung der Formel (E);
Peak N4: Verbindung der Formel (F);
Peaks N1, N2, N5-N10 wurden nicht weiter untersucht.
Laufmittel: $CH_3CN$ / $H_2O$ = 65 : 35 (v/v); 1 ml/min; Detektion: UV 206 nm.

FIG. 4 : GC-Diagramm des derivatisierten Oligosaccharid-Alditol-Gemisches (Trifluoracetate der Methylglycoside) aus Axolotl mexicanum. Das Oligosaccharid-Gemisch wird durch Methanolyse aus dem Lyophilisat der Schleimhülle von Eiern von Axolotl mexicanum erhalten. Die Probenvorbereitung erfolgt nach Zanetta et al., 1972 (J. Chromatogr. 69, 291). Säule: Chromosorb Q, Silicon 5 %, OV 101 (1m x 0.32 cm), Varian GC. Fuc, Gal, GlcNAc, GalNAc und KDN haben die oben angegebenen Bedeutungen.

Soweit nicht anderweitig angegeben, werden bei der Isolierung, Aufreinigung, Charakterisierung und Herstellung der erfindungsgemäßen Verbindungen und Mittel gängige, literaturbekannte Standardmethoden angewandt.

Amphibien, wie Molche, Frösche oder Kröten sind weit verbreitet und entweder direkt in ihrer natürlichen Umgebung oder aber in Aquarien im einschlägigen Fachhandel zugänglich. Dasselbe gilt auch für den Laich dieser Tiere, aus dem die benötigten Eier gewonnen werden. Es bestehen also keinerlei Probleme, an das zur Durchführung der Erfindung notwendige Ausgangsmaterial, nämlich die Schleimhülle der Eier, heranzukommen.

Im folgenden wird die Erfindung in allgemeiner Form beschrieben.

Herstellung der Verbindungen der Formel (A) bis (D):

Man präpariert den Laich der Amphibienart Pl,waltlii in der Weise, daß man das eigentliche Ei aus der dicken es umgebenden Schleimhülle vorzugsweise mechanisch entfernt. Das gallertartige Material wird lyophilisiert und das trockene Material der $\beta$ – Eliminierung unterworfen.

Die $\beta$ – Eliminierung dient dazu, Glycoproteine mit vorzugsweise O – glycosidischer Bindung zu spalten (z. B. Downs et al. 1977, Int. J. Peptide Protein Res. 10, 315; Aminoff et al., 1980, Anal. Biochem. 101, 44). Dabei wird das lyophilisierte Material in 50 bis 100 mM alkalischer Lösung, beispielsweise KOH oder NaOH, vorzugsweise NaOH, mit einem Wasserstoff übertragenden Reduktionsmittel, vorzugsweise $NaBH_4$ oder $KBH_4$, insbesondere aber $NaBH_4$, bei Temperaturen zwischen 20 und 45 ° C, vorzugsweise 35 – 40 ° C, etwa 20 – 50 Stunden, vorzugsweise 24 – 30 Stunden lang umgesetzt (Carlson 1966, J. Biol. Chem 241, 2984).

Die Spaltung eventuell vorliegender N – glycosidischer Bindungen unter diesen Bedingungen ist nicht vollständig auszuschließen (z.B. Anal. Biochem. (1982), 119, 351).

Die angebenen Bedingungen bewirken, daß der Zuckerrest, an den der Proteinrest gebunden ist – bei den vorliegenden erfindungsgemäßen Verbindungen ist dies der Rest X – in seine offenkettige Form (Aldehyd – Form) überführt wird und durch die reduktiven Bedingungen zu dem entsprechenden Zuckeralkohol umgesetzt wird, während die anderen Zuckerreste unverändert und in ihrer ursprünglichen Ringform erhalten bleiben.

Die Reaktion wird vorzugsweise bei tiefen Temperaturen (0 – 5 ° C) und Zugabe eines Kationenaustauschers beispielsweise Dowex[R] (Biorad) gestoppt und der pH – Wert anschließend auf 5 – 6 eingestellt. Aus der vorzugsweise filtrierten Lösung wird die entstandene Borsäure beispielsweise in Form ihres Methylesters abdestilliert und die Lösung anschließend nach Standardmethoden entsalzt.

Die reduzierten Glycane werden erfindungsgemäß mittels präparativer oder semi – präparativer HPLC aufgetrennt. Anderweitige Auftrennung, beispielsweise mittels Gelfiltration ist ebenfalls durchführbar.

Als Trägermaterial für die HPLC eignet sich Kieselgel, welches eine aminierte Phase aufweist. Bevorzugt ist eine Aminopropylsilyl – Phase. Letztlich sind aber alle Träger und Phasen geeignet, die zur Trennung von Zucken und Zuckerderivaten bekannt sind. Als Elutionsmittel werden bevorzugt Acetonitril/Puffer – bzw. Acetonitril/Wasser – Gemische eingesetzt.

Man erhält vier Fraktionen, wobei drei in reiner Form auftreten (Verbindungen der Formeln (A), (B) und (D)) und die vierte Fraktion in zwei isomeren Formen vorkommt, wobei die Hauptkomponente die Struktur (C) aufweist (FIG. 1.).

Die Charakterisierung der erfindungsgemäßen Substanzen erfolgt nach Standardmethoden vorzugsweise mittels Gaschromatographie (Zanetta et al. 1972, J. Chromatogr. 69, 291) (FIG. 2), Papierchromatographie, HPLC, Nuclear Magnetic Resonance (NMR) Spektroskopie, Dünnschichtchromatographie und Massenspektrometrie (siehe Beispiele).

Erfindungsgemäß kann die Glycoproteinspaltung des lyophilisierten Materials unter schwach alkalischen Bedingungen vorgenommen werden. Die schwach alkalische Hydrolyse wird z. B. mit 0.5 – 10 mM NaOH durchgeführt unter sonst analogen Bedingungen, wie oben angegeben, aber ohne Zusatz eines Reduktionsmittels. In diesem Fall entstehen die nativen (nicht reduzierten) Glycane, in denen die Ringstruktur vollständig erhalten geblieben ist.

Die Auftrennung der nativen Glycane erfolgt in gleicher Weise und mit den selben Mitteln, wie es bereits für die entsprechenden Alditole beschrieben wurde. Man erhält vergleichbare chromatographische Diagramme (FIG.1 u. 2), in denen die jeweiligen Retentionszeiten nur geringfügig verändert sind.

Herstellung der Verbindungen der Formel (E) bis (I):

Man präpariert den Laich der Amphibienart A. mexicanum in der Weise, daß man das eigentliche Ei aus der dicken es umgebenden Schleimhülle vorzugsweise mechanisch entfernt. Das gallertartige Material wird lyophilisiert und das trockene Material der $\beta$ – Eliminierung unterworfen.

Die reduktive $\beta$ – Eliminierung des Mucins aus Axolotl wird analog wie oben beschrieben, durchgeführt. Bei der $\beta$ – Eliminierung enthält man nach der Entsalzung zunächst drei unterschiedliche uneinheitliche Fraktionen. Eine Fraktion (ca. 7 – 8 % des eingesetzten lyophilisierten Materials) enthält lediglich hochmolekulare Bestandteile und wird nicht weiter verwendet. Fraktion 2 (ca. 4 – 5 %), welche saure Komponenten enthält, kann sofort der chromatographischen Aufreinigung unterzogen werden. Man erhält nach HPLC die Peaks A1 bis A6 aus FIG. 3(a). Dabei entsprechen die Peaks A3, A5 und A6 den Verbindungen der Formeln (G), (H) und (I). Die dritte Fraktion (ca. 15 – 20 %) enthält neutrale und saure Komponenten und wird vorzugsweise über einen Anionenaustauscher weiter aufgereinigt, ehe eine weitere Entsalzung sich anschließt.

Man erhält aus der neutralen Fraktion nach HPLC die Peaks N1 – N10, wobei die Peaks N3 und N4 den Verbindungen (E) und (F) entsprechen. Die anderen Peaks aus der zweiten und dritten Fraktion wurden erfindungsgemäß nicht weiter charakterisiert bzw. verwendet, ebenso wie die sauren Komponenten aus der dritten Fraktion.

Die Methoden und Mittel der Aufreinigung und Analytik entsprechen denen, wie oben für die Aufreinigung der Oligosaccharide aus Pleurodeles angegeben wurde.

Herstellung der KDN – freien Oligosaccharide der Formeln (A) – (I),

Herstellung von KDN:

Die Abspaltung des KDN – Restes erfolgt erfindungsgemäß vorzugsweise aus den freigesetzten Oligosacchariden oder deren Alditolen. Die Bedingungen werden so gewählt, daß lediglich der KDN – Rest von dem Oligosaccharid abgespalten wird, während die anderen Reste Fuc, Gal, GlcNac oder GalNAc gebunden bleiben. Die Abspaltung wird vorzugsweise mit schwachen anorganischen oder organischen Säuren, beispielsweise mit Ameisensäure bei Temperaturen zwischen 40 und 120 °C durchgeführt. Die Abtrennung von KDN bzw. den desialylierten Oligosacchariden bzw. deren Alditolen kann beispielsweise mittels Gelfiltration nach Standardmethoden auf einfache Weise bewerkstelligt werden.

Herstellung von Neoglycoproteinen/peptiden:

Die erfindungsgemäßen Oligosaccharide (A) – (I), die in der nativen, nicht reduzierten Form vorliegen, können nun nach Standardmethoden an geeignete Proteine oder Peptide gekoppelt werden, wodurch sie ihre immunogenen Eigenschaften entfalten.

Eine Reihe von geeigneten Kopplungsmethoden ist in "Advances in Carbohydrate Chemistry", 1980, Vol. 37, 225 zusammengestellt. Vorzugsweise werden die Konjugate der Oligosaccharide (A) bis (I) nach der Methode von Gray (Arch. Biochem. Biophys. 163, (1984), 426) hergestellt. Dabei wird eine Aminogruppe des Proteins oder Peptides unter Einwirkung von $BH_3CN^-$ an das O – glycosidische C – Atom des Oligosaccharides (Rest X der Verbindungen (A) – (I), worin X vorzugsweise GalNac bedeutet) gebunden. Selbstverständlich können entsprechende Konjugate auch mittels anderer bekannten Methoden der Kohlenhydrat – bzw. Proteinchemie hergestellt werden, so beispielsweise durch Einführung geeigneter Schutzgruppen in den Kohlenhydratrest.

Als Proteine eignen sich beispielsweise Human – oder Rinder – Serumalbumin (HSA, BSA), KLH (Keyhole Limpet haemocyanin), Cholera – Toxin oder Tetanus – Toxin.

Das Protein bzw. Peptid kann auch über einen Spacer oder Linker an eines der erfindungsgemäßen Oligosaccharide gekoppelt sein. Als Linker eignen sich beispielsweise Verbindungen des Typs $CH_2OH-(CH_2)_n-COOR$, worin R Alkyl mit 1 bis 4 C – Atomem und n eine ganze Zahl von 4 bis 12, vorzugsweise 8 bedeuten.

Derartige Konjugate werden z. B. nach Lemieux et al. (J. Am. Chem. Soc. 97, (1975), 4076, bzw. Can. J. Biochem. 55, (1977), 507) hergestellt. Dabei reagiert zunächst das Oligosaccharid mit dem Linker, dessen Ester – Gruppe über eine Hydrazid – in eine Azid – Gruppe überführt wird. Letztere wird schließlich mit dem gewünschten Peptid bzw. Protein umgesetzt.

Herstellung von Neoglycolipiden:

Die erfindungsgemäßen Oligosaccharide können auch an Lipide nach Standardmethoden gekoppelt werden. Hierbei kann vorteilhaft die reduktive Aminierung nach Wang et al. (Anal. Biochem. 144 (1984), 366) eingesetzt werden. Bei dieser Methode wird der eigentliche Lipidrest über eine funktionelle Gruppe eingeführt. Als Reagens verwendet man beispielsweise das im Handel erhältliche Hexadecylanilin. Entsprechend lassen sich auch andere Lipidreste an Anilin binden. Die Reduktion des intermediär gebildeten Imids erfolgt vorzugsweise mit $BH_3CN^-$.Diese Methode ist besonders geeignet bei Neokonjugaten, die einen Sialyl – Rest (KDN) enthalten.

Herstellung von Immunadsorbentien

Die erfindungsgemäß hergestellten Oligosaccharide können zur Herstellung wirksamer monospezifischer Immunadsorbentien verwendet werden. Derartige Immunadsorbentien haben eine große Bedeutung z.B. bei der Entfernung von unerwünschten Antikörpern aus Blutseren und zur Isolierung und Reinigung

spezifischer Antikörper durch Adsorptions–Desorptionsmethoden. In diesen Fällen ist der die antigene Determinante enthaltene Zucker fest mit einem Träger verbunden.

In anderen Fällen können Antikörper an einen Träger kovalent (z.B. $CNBr^-$ aktivierte Sepharose Cl–4B, Pharmacia; Affi–Gel 10/15/102, Biorad) oder nicht–kovalent (z.B. Staphylococcal protein A–Sepharose Cl–4B, SPA–Seph$^R$, Pharmacia) gebunden werden, so daß sich entsprechende antigene Determinanten in Zuckergemischen an derartigen Konjugaten reinigen bzw. auftrennen lassen.

Als Trägerstoffe für Immunoadsorbentien sind beispielsweise geeignet: aminierte Sepharose, aminiertes Polystyrol, Polyvinylamin, aminierter Polyvinylalkohol, aminiertes Polyvinylacrylamid, Aminoethylcellulose, aminierte Carboxymethylcellulose, aminierte Carboxymethylagarose, aminiertes Glas oder aminiertes Kieselgel. Diese Stoffe können als Körperchen oder Latexpartikel verwendet werden, sind jedoch auch an Oberflächen von Platten, Röhren, Schläuchen etc. aus anorganischen oder organischen Materialien einsetzbar.

Die Herstellung entsprechender Immunadsorbentien geschieht nach Standardmethoden, z.B. nach Erickson et al. (Biochem. Biophys. Res. Commun. 43, 1164 (1971)) oder gemäß der EP–OS–0 044 188 oder der US 4,238,473 oder nach Lemieux (Chem Soc. Rev. 7, (1978), 423) oder nach Mazid et al. (Bioconjugate Chem. 2, (1991), 32).

Bestimmung der Immunogenität in vitro

Erfindungsgemäß wird ein in–vitro Immunoassay zur Verfügung gestellt. Danach werden menschliche Leukocyten nach bekannten Methoden isoliert und gereinigt und mit einem erfindungsgemäßen Neoglycokonjugat, vorzugsweise Neoglycoprotein/peptid, inkubiert. Hierdurch werden die Lymphocyten zur verstärkten Proliferation stimuliert. Diese verstärkte Stimulation läßt sich durch den erhöhten Einbau an zugesetztem radioaktiv markiertem Thymidin auf einfache Weise messen. Man erhält erfindungsgemäß eine erhöhte Proliferation zwischen 40 und 65 %, je nachdem welches Neoglycokonjugat eingesetzt wurde.

Beispiel 1:

160 mg der lyophilisierten Schleimhülle aus Pleurodeles waltlii werden in 50 mM NaOH und 1.0 M $NaBH_4$ (10 ml) bei 37 °C 24 Stunden lang im Dunkeln und unter Rühren umgesetzt. Man stoppt die Reaktion bei ca. 4 °C durch Zugabe von Dowex$^R$ 50 x 8 (25–50 mesh; $H^+$–Form; Fa. Biorad), wobei sich ein pH–Wert von etwa 4.5 einstellt. Die Lösung wird filtriert, mit 0.1 M NaOH auf pH 5.5 eingestellt und konzentriert. Die entstandene Borsäure wird als Methylester durch mehrmalige Zugaben von Methanol abdestilliert. Anschließend erfolgt eine Entsalzung über eine Bio–Gel P2–Säule (2 x 50 cm, Pharmacia).

Beispiel 2:

Die nach Beispiel 1 hergestellten Oligosaccharid–Alditole werden durch HPLC aufgetrennt. Als Trägermaterial dient eine mit einer Aminopropylsilyl–Phase beschichteten Kieselgelsäule (Supelcosil$^R$ LC–$NH_2$, Fa. Supelco, Schweiz). Das Gemisch wird mit Acetonitril / $KH_2PO_4$ (30 mM, pH 7.0) = 65 / 35 eluiert (1 ml/min). Die Detektion erfolgt im UV bei 206 nm. Man erhält die 4 Peaks der FIG. 1. Jede Fraktion ergibt etwa 6 mg Reinsubstanz.
Peak I: Verbindung der Formel (A);
Peak II: Verbindung der Formel (B);
Peak III: Verbindung der Formel (C);
Peak IV: Verbindung der Formel (D).

Die Analytik wird mittels Gaschromatographie (FIG. 2) und $^1H$– und $^{13}C$– NMR–Spektroskopie durchgeführt (BRUKER AM–400 WB Spektrometer). In den Tabellen 1 und 2 sind die chemischen Shifts angegeben (durch Referenzmessung zu Aceton (in $D_2O$, bei 27 °C) bzw. DSS). Die Verbindungen der Formeln (A) bis (D) liegen in Form ihrer Alditole vor (X = GalNAc–ol).

Tab. 1: $^1H$ NMR-Shifts für die Verbindungen (A) bis (D).

| | H-1 | H-2 | H-3 | H-4 | H-5 | H-6 | H-7 | H-8 | H-9 | $CH_3$ |
|---|---|---|---|---|---|---|---|---|---|---|
| KDN(α2-6) | - | - | 1.652(ax)<br>2.676(eq) | 3.603 | 3.524 | 3.660 | n.d. | n.d. | n.d. | - |
| Gal(β1-4) | 4.444 | 3.494 | 3.660 | 3.902 | 3.62 | 3.77<br>3.72 | - | - | - | - |
| Fuc(α1-3) | 5.132 | 3.700 | 3.902 | 3.792 | 4.818 | 1.176 | - | - | - | - |
| GlcNAc(β1-3) | 4.645 | 3.981 | 3.897 | 4.950 | 3.603 | 4.020<br>3.858 | - | - | - | 2.066 |
| GalNAc-ol | 3.612<br>3.586 | 4.244 | 3.978 | 3.634 | 4.161 | 3.814<br>3.474 | - | - | - | 2.023 |
| | | | | | | | | | | |
| KDN(α2-6) | - | - | 1.655(ax)<br>2.675(eq) | 3.603 | 3.524 | 3.669 | n.d. | n.d. | n.d. | - |
| Fuc(α1-2) | 5.276 | 3.805 | 3.774 | 3.836 | 3.256 | 1.273 | - | - | - | - |
| Gal(β1-4) | 4.491 | 3.651 | 3.858 | 3.871 | 3.61 | 3.77<br>3.72 | - | - | - | - |
| Fuc(α1-3) | 5.113 | 3.699 | 3.915 | 3.810 | 4.871 | 1.236 | - | - | - | - |
| GlcNAc(β1-3) | 4.631 | 3.96 | n.d. | n.d. | 3.467 | 4.034<br>3.853 | - | - | - | 2.065 |
| GalNAc-ol | 3.630<br>3.568 | 4.247 | 3.971 | 3.599 | 4.184 | 3.814<br>3.474 | - | - | - | 2.024 |
| | | | | | | | | | | |
| KDN(α2-6) | - | - | 1.654(ax)<br>2.674(eq) | 3.599 | 3.520 | 3.664 | 3.858 | 3.893 | 3.92<br>3.83 | - |
| GlcNAc(α1-3) | 5.198 | 4.242 | 3.924 | 3.998 | 4.227 | 3.757 | - | - | - | 2.035 |
| Fuc(α1-2) | 5.297 | 3.783 | 3.713 | 3.849 | 4.336 | 1.303 | - | - | - | - |
| Gal(β1-4) | 4.552 | 3.871 | 3.941 | 4.200 | 3.577 | 3.75<br>3.70 | - | - | - | - |
| Fuc(α1-3) | 5.128 | 3.699 | 3.915 | 3.788 | 4.871 | 1.274 | - | - | - | - |
| GlcNAc(β1-3) | 4.634 | 3.96 | n.d. | 3.937 | 3.467 | 4.053<br>3.858 | - | - | - | 2.066 |
| GalNAc-ol | 3.625<br>3.555 | 4.246 | 3.970 | 3.599 | 4.180 | 3.810<br>3.474 | - | - | - | 2.024 |

Tab. 2: $^{13}C$ NMR-Shifts für die Verbindung (D).

| | C-1 | C-2 | C-3 | C-4 | C-5 | C-6 | C-7 | C-8 | C-9 | CO | $CH_3$ |
|---|---|---|---|---|---|---|---|---|---|---|---|
| KDN(α2-6) | 175.07 | 101.76 | 41.05 | 71.54 | 71.27 | 74.88 | 69.25 | 73.39 | 64.08 | - | - |
| GalNAc(α1-3) | 92.73 | 50.92 | 70.48 | 69.86 | 72.32 | 62.56 | - | - | - | 175.72 | 23.31 |
| Fuc(α1-2) | 100.18 | 69.10 | 71.34 | 73.08 | 68.26 | 16.92 | - | - | - | - | - |
| Gal(β1-4) | 101.53 | 74.16 | 77.27 | 63.87 | 76.05 | 62.69 | - | - | - | - | - |
| Fuc(α1-3) | 99.83 | 69.10 | 69.25 | 73.39 | 68.19 | 16.74 | - | - | - | - | - |
| GlcNAc(β1-3) | 103.06 | 57.83 | n.d. | 75.19 | 76.43 | 61.46 | - | - | - | 176.23 | 23.70 |
| GalNAc-ol | 61.41 | 52.77 | 77.84 | 70.81 | 69.39 | 66.77 | - | - | - | 175.07 | 23.41 |

Beispiel 3:

Die nach Beispiel 2 erhaltenen Oligosaccharide bzw. deren Alditole werden nach Abziehen des Eluens in Wasser aufgenommen und mit Ameisensäure bis zu einer Endkonzentration von 0.1 M versetzt. Die Lösung wird ca. 1 Stunde lang bei 100 ° C inkubiert, anschließend wird mit 0.1 M NaOH neutralisiert. Die

Lösung wird schließlich über eine Sephadex G-25- Säule gegeben. Man erhält in nahezu quantitativer Ausbeute die desialylierten Oligosaccharide der Formeln (A)-(D) sowie KDN (3-Deoxy-D-glycero-D-galacto-nonulosonsäure).

Beispiel 4:

1.9 g der lyophilisierten Schleimhülle aus Axolotl mexicanum werden in 50 mM NaOH und 1.0 M $NaBH_4$ (300 ml) bei 37 °C 24 Stunden lang im Dunkeln und unter Rühren umgesetzt. Man stoppt die Reaktion bei ca. 4 °C durch Zugabe von Dowex[R] 50x8 (25-50 mesh; $H^+$-Form; Fa. Biorad), wobei sich ein pH-Wert von etwa 4.5 einstellt. Die Lösung wird filtriert (Filtrat ca. 1.3 g), mit 0.1 M NaOH auf pH 5.5 eingestellt und konzentriert. Die entstandene Borsäure wird als Methylester durch mehrmalige Zugaben von Methanol abdestilliert. Anschließend erfolgt eine Entsalzung über eine Bio-Gel P4-Säule (2 x 80 cm, Pharmacia).

Man erhält drei Fraktionen. Fraktion (Peak) I der P4-Säule enthält nur hochmolekulare Verbindungen (123 mg) und wird verworfen. Fraktion (Peak) II enthält saure Komponenten (80 mg) und wird direkt der HPLC-Aufreinigung unterworfen (Beispiel 5). Fraktion (Peak) III enthält saure und neutrale Verbindungen (320 mg). Das Gemisch wird auf eine Dowex[R] 1 x 2-Säule (Biorad, mesh 200-400; $HCOO^-$ -Form, 2 x 15 cm) gegeben. Die neutralen Komponenten werden mit Wasser, die sauren Komponenten mit Pyridin-Acetat Puffer (40 mM) von der Säule gewaschen.

Beispiel 5:

Die nach Beispiel 4 hergestellten Oligosaccharid-Alditole werden durch HPLC aufgetrennt. Als Trägermaterial dient eine mit einer Aminopropylsilyl-Phase beschichteten Kieselgelsäule (Supelcosil[R] LC-$NH_2$, Fa. Supelco, Schweiz). Das Gemisch der sauren Verbindungen wird mit Acetonitril / $KH_2PO_4$ (30 mM, pH 7) = 65 / 35, das Gemisch der neutralen Verbindungen mit Acetonitril / Wasser = 65 / 35 eluiert (1 ml/min). Die Detektion erfolgt im UV bei 206 nm.

Die sauren Verbindungen der Fraktion III (Beispiel 4) werden erfindungsmäßig nicht weiter verwendet.

Die sauren Verbindungen der Fraktion II ergeben das HPLC-Diagramm der FIG. 3(a). Dabei entsprechen die Peaks A3, A5 und A6 den erfindungsgemäßen Verbindungen (G), (H) und (I).

Die neutralen Verbindungen der Fraktion III ergeben das HPLC-Diagramm der FIG. 3(b).

Peak N3: Verbindung der Formel (E);

Peak N4: Verbindung der Formel (F);

Peaks N1, N2, N5-N10 werden nicht weiter untersucht.

Peak N2 entspricht der bekannten Struktur GlcNAc(β1-3)-GalNAc-ol.

Die Analytik wird mittels Gaschromatographie (Fig. 4) und $^1H$- und $^{13}C$- NMR-Spektroskopie durchgeführt (BRUKER AM-400 WB Spektrometer). In den Tabellen 1 und 2 sind die chemischen Shifts angegeben (durch Referenzmessung zu Aceton (in $D_2O$, bei 27 °C) bzw. DSS). Die Verbindungen (E) bis (I) liegen in Form ihrer Alditole (X = GalNAc-ol) vor.

Die Tabellen 3-5 enthalten Daten über $^1H$ und $^{13}C$ NMR-Shifts für die Verbindungen (G)-(I).

Tab. 3: $^{1}H$ und $^{13}C$ NMR-Shifts für die Verbindung (G)

| | H-1 | H-2 | H-3 | H-4 | H-5 | H-6 | H-7 | H-8 | H-9 | NAc | |
|---|---|---|---|---|---|---|---|---|---|---|---|
| Fuc(α1-3) | 5.073 | 3.805 | 3.959 | 3.827 | 4.293 | 1.216 | - | - | - | - | |
| Fuc(α1-4) | 5.174 | 3.897 | 3.930 | 4.034 | 4.155 | 1.216 | - | - | - | - | |
| KDN(α2-6) | - | - | 1.786ax<br>2.805eq | 3.638 | 3.727 | 3.727 | 3.876 | 3.902 | N.D. | - | |
| Fuc(α1-2) | 5.305 | 3.810 | 3.796 | 3.827 | 4.217 | 1.232 | - | - | - | - | |
| Gal(β1-4) | 4.529 | 3.665 | 3.876 | 3.893 | 3.700 | 3.900 | - | - | - | - | |
| GlcNAc(β1-3) | 4.611 | 3.801 | 3.700 | 3.793 | 3.480 | 3.994<br>3.823 | - | - | - | 2.077 | |
| GalNAc-ol | 3.630<br>3.577 | 4.266 | 3.978 | 3.577 | 4.210 | 3.814 | - | - | - | 2.033 | |
| | C-1 | C-2 | C-3 | C-4 | C-5 | C-6 | C-7 | C-8 | C-9 | $CH_3$ | CO |
| Fuc(α1-3) | 96.66 | 69.33 | 70.77 | 72.98 | 68.25 | 16.63 | - | - | - | - | - |
| Fuc(α1-4) | 101.40 | 68.10 | 75.95 | 69.54 | 67.22 | 16.63 | - | - | - | - | - |
| KDN(α2-6) | 174.95 | 101.73 | 40.13 | 80.05 | 70.17 | 74.65 | 69.23 | 73.29 | 64.10 | - | - |
| Fuc(α1-2) | 100.69 | 69.52 | 70.95 | 73.22 | 68.25 | 16.63 | - | - | - | - | - |
| Gal(β1-4) | 101.60 | 77.67 | 74.87 | 70.45 | 76.53 | 62.40 | - | - | - | - | - |
| GlcNAc(β1-3) | 103.60 | 57.02 | 73.27 | 77.47 | 76.25 | 61.66 | - | - | - | 23.62 | 176.19 |
| GalNac-ol | 61.48 | 52.68 | 77.99 | 70.64 | 69.33 | 66.86 | - | - | - | 23.42 | 175.53 |

Tab. 4: $^{1}H$ und $^{13}C$ NMR-Shifts für die Verbindung (H)

| | H-1 | H-2 | H-3 | H-4 | H-5 | H-6 | H-7 | H-8 | H-9 | NAc | |
|---|---|---|---|---|---|---|---|---|---|---|---|
| Fuc(α1-4) | 5.147 | 3.770 | 3.888 | 3.823 | 4.161 | 1.242 | - | - | - | - | |
| KDN(α2-6) | - | - | 1.772ax<br>2.782eq | 3.638 | 3.708 | 3.717 | 3.866 | 3.893 | 3.94<br>3.69 | - | |
| Fuc(α1-2) | 5.314 | 3.794 | 3.802 | 3.823 | 4.206 | 1.236 | - | - | - | - | |
| Gal(α1-4) | 4.958 | 3.845 | 3.919 | 4.029 | 4.351 | 3.700 | - | - | - | - | |
| Gal(β1-4) | 4.577 | 3.700 | 3.936 | 4.029 | 3.770 | 3.70 | - | - | - | - | |
| GlcNAc(β1-3) | 4.613 | 3.788 | 3.700 | 3.780 | 3.485 | 4.024<br>3.845 | - | - | - | 2.079 | |
| GalNAc-ol | 3.625<br>3.577 | 4.266 | 3.980 | 3.586 | 4.206 | 3.814<br>3.485 | - | - | - | 2.030 | |
| | C-1 | C-2 | C-3 | C-4 | C-5 | C-6 | C-7 | C-8 | C-9 | $CH_3$ | CO |
| Fuc(α1-4) | 101.33 | 69.69 | 70.83 | 72.97 | 68.23 | 16.61 | - | - | - | - | - |
| KDN(α2-6) | 174.95 | 101.73 | 40.10 | 79.69 | 70.31 | 74.69 | 69.21 | 73.29 | 64.10 | - | - |
| Fuc(α1-2) | 101.00 | 69.69 | 70.95 | 73.21 | 68.45 | 16.67 | - | - | - | - | - |
| Gal(α1-4) | 101.91 | 69.83 | 70.42 | 70.28 | 72.35 | 61.75 | - | - | - | - | - |
| Gal(β1-4) | 102.18 | 77.85 | 74.36 | 79.85 | 76.64 | 61.90 | - | - | - | - | - |
| GlcNAc(β1-3) | 103.56 | 57.18 | 74.36 | 78.09 | 76.23 | 61.54 | - | - | - | 23.62 | 176.20 |
| GalNAc-ol | 61.49 | 52.68 | 78.00 | 70.68 | 69.34 | 66.83 | - | - | - | 23.42 | 175.52 |

Tab. 5: $^{1}H$ und $^{13}C$ NMR-Shifts für die Verbindung (I)

| | H-1 | H-2 | H-3 | H-4 | H-5 | H-6 | H-7 | H-8 | H-9 | NAc | |
|---|---|---|---|---|---|---|---|---|---|---|---|
| Fuc(α1-3) | 5.073 | 3.807 | 3.961 | 3.824 | 4.294 | 1.215 | - | - | - | - | |
| Fuc(α1-4) | 5.174 | 3.908 | 3.930 | 4.040 | 4.154 | 1.266 | - | - | - | - | |
| KDN(α2-6) | - | - | 1.787ax<br>2.806eq | 3.644 | 3.728 | 3.723 | 3.872 | 3.899 | 3.916<br>3.741 | - | |
| Fuc(α1-2) | 5.314 | 3.794 | 3.802 | 3.824 | 4.206 | 1.236 | - | - | - | - | |
| Gal(α1-4) | 4.957 | 3.842 | 3.917 | 4.031 | 4.350 | 3.706 | - | - | - | - | |
| Gal(β1-4) | 4.576 | 3.698 | 3.934 | 4.031 | 3.767 | 3.706 | - | - | - | - | |
| GlcNAc(β1-3) | 4.613 | 3.798 | 3.706 | 3.784 | 3.842 | 4.022<br>3.851 | - | - | - | 2.078 | |
| GalNAc-ol | 3.631<br>3.579 | 4.268 | 3.982 | 3.579 | 4.215 | 3.820<br>3.486 | - | - | - | 2.030 | |
| | C-1 | C-2 | C-3 | C-4 | C-5 | C-6 | C-7 | C-8 | C-9 | $CH_3$ | CO |
| Fuc(α1-3) | 96.67 | 69.33 | 70.77 | 72.97 | 68.24 | 16.62 | - | - | - | - | - |
| Fuc(α1-4) | 101.40 | 68.09 | 75.95 | 69.55 | 67.91 | 16.62 | - | - | - | - | - |
| KDN(α2-6) | 174.94 | 101.72 | 40.14 | 80.06 | 70.17 | 74.66 | 69.22 | 73.30 | 64.09 | - | - |
| Fuc(α1-2) | 101.01 | 69.71 | 70.94 | 73.21 | 68.46 | 16.67 | - | - | - | - | - |
| Gal(α1-4) | 101.91 | 69.83 | 70.41 | 70.28 | 72.34 | 61.75 | - | - | - | - | - |
| Gal(β1-4) | 102.19 | 77.88 | 74.35 | 79.84 | 76.63 | 61.89 | - | - | - | - | - |
| GlcNAc(β1-3) | 103.56 | 57.19 | 73.21 | 78.10 | 76.23 | 61.54 | - | - | - | 23.62 | 176.19 |
| GalNAc-ol | 61.48 | 52.68 | 77.98 | 70.64 | 69.33 | 66.87 | - | - | - | 23.42 | 175.53 |

Tab. 6: $^1$H NMR-Shifts für die Verbindungen (E) (= Peak N3), (F) (= Peak N4) und Peak N2.

| Residue | | N-2 | N-3 | N-4 |
|---|---|---|---|---|
| GalNAc-ol | H-2 | 4.286 | 4.283 | 4.281 |
| | H-3 | 3.995 | 3.989 | 3.991 |
| | H-4 | 3.547 | 3.549 | 3.549 |
| | H-5 | 4.139 | 4.143 | 4.139 |
| | NAc | 2.037 | 2.037 | 2.037 |
| GlcNAc(β1-3) | H-1 | 4.604 | 4.610 | 4.613 |
| | NAc | 2.084 | 2.087 | 2.086 |
| | H-5 | 3.474 | 3.499 | 3.501 |
| | H-6 | 3.949 | 4.010 | - |
| Gal(β1-4) | H-1 | - | 4.523 | 4.569 |
| | H-4 | - | 3.895 | 4.026 |
| Fuc(α1-2) | H-1 | - | 5.310 | 5.317 |
| | H-5 | - | 4.220 | 4.206 |
| | H-6 | - | 1.233 | 1.236 |
| Gal(α1-4) | H-1 | - | - | 4.954 |
| | H-4 | - | - | 4.026 |
| | H-5 | - | - | 4.347 |

Beispiel 6:

Die nach Beispiel 2 erhaltenen Oligosaccharide bzw. deren Alditole werden nach Abziehen des Eluens in Wasser aufgenommen und mit Ameisensäure bis zu einer Endkonzentration von 0.1 M versetzt. Die Lösung wird ca. 3 Stunden bei 100 °C inkubiert, anschließend wird mit 0.1 M NaOH neutralisiert. Die Lösung wird schließlich über eine Sephadex G-25- Säule gegeben. Man erhält in nahezu quantitativer Ausbeute die desialylierten Oligosaccharide der Formeln (A)-(D) sowie KDN (3-Deoxy-D-glycero-D-galacto-nonulosonsäure).

Beispiel 7:

300 µmol der gereinigten Verbindung der Formel (B), worin X GalNAc ist, und 1 µmol Rinderserumalbumin werden in 2.5 ml 0.2 M Phosphatpuffer (pH 8.0) + 1 Tropfen Toluol gelöst. Man gibt 1.6 mmol $NaBH_3CN$ hinzu und inkubiert ca. 7 Tage lang bei 37 °C unter ständigem Rühren. Anschließend wird ungebundenes Oligosaccharid und der Überschuß an Reagens durch Filtration durch eine Membranfilter (PM 10, Millipore) abgetrennt, wobei das Konjugat durch intensives häufiges Waschen mit Wasser erhalten wird.

Der Reaktionsverlauf wird mittels Gaschromatographie verfolgt.

Beispiel 8:

Analog Beispiel 7 wird ein Glycokonjugat aus dem Oligosaccharid der Formel (E) und humanem Serumalbumin hergestellt.

Beispiel 9:

Aus Verbindung (C) und 8-Methoxycarbonyloctanol wird gemäß der Vorschrift von Lemieux et al. (l. c.) das entsprechende 8-Methoxycarbonyl-octyl-Saccharid und daraus mit Hydrazin in Ethanol die entsprechende Hydrazid-Verbindung hergestellt. 100 µmol der trockenen, hydrazinfreien Verbindung werden in 1.5 ml trockenem DMF aufgenommen und 400 µmol von 3.6 M HCl in wasserfreiem Dioxan hinzugefügt. Die Lösung wird auf ca. -20 °C abgekühlt und mit 140 µmol t-Butylnitrit in 100 µl DMF versetzt. Nach 30 Minuten werden 40 µmol Sulfaminsäure in DMF (100 µl) hinzugefügt und die Temperatur auf -30 °C gesenkt. 2 µmol Rinderserumalbumin werden gelöst in einer Pufferlösung (25 mg Protein / ml Puffer), bestehend aus 0.35 M $KHCO_3$ in 0.08 M $Na_2B_4O_7$ (pH 9.0), und zu der azidhaltigen Lösung hinzugegeben. Man erhält das entsprechende, über den Linker $-O-(CH_2)_8-CO-$ gebundene Neoglycokonjugat.

Beispiel 10:

Analog Beispiel 9 wird ein Glycokonjugat aus dem Oligosaccharid der Formel (I) und humanem Serumalbumin hergestellt.

Beispiel 11:

5 mg des nativen Oligosaccharides der Formel (D) werden in 50 µl Wasser gelöst, und es werden 250 µl einer Hexadecylanilin-Lösung (36 mg in 250 µl Chloroform) hinzugefügt. Man inkubiert die Lösung 2 Stunden lang bei 50 °C und fügt anschließend 250 µl einer Natriumcyanoborhydrid-Lösung (25 mg in 250 µl Methanol) hinzu. Die Lösung wird 16 Stunden lang bei 37 °C inkubiert und unter Stickstoff zur Trockene eingeengt. Der Rückstand wird in alkalischem Wasser (pH 9 mit $NH_3$) aufgenommen und es wird mehrmals mit jeweils 1 ml Chloroform extrahiert. Das Neoglycolipid ist in der wäßrigen Phase. Die Reaktion wird mittels TLC kontrolliert: Kiesel-Gel 60 (Fa. Merck, FRG), Chloroform / Methanol / Wasser = 130 / 50 / 9, Anfärbung mit Orcinol / $H_2SO_4$.

Beispiel 12:

Poröse Glaskörperchen (0,075-0,15 mm) werden unter Verwendung von 3-Aminopropyltriäthoxysilan unter Standardbedingungen (Westphal et al., 1974, Methods of Enzymology 34(b),64) aminiert. Der Amingehalt beträgt 75 µmol/g, bestimmt nach der Methode von Esko et al. (Acta Chem. Scand. 22 (1968), 3342).

Die Verbindung der Formel (F) in der Ringform oder in der offenkettigen Aldehydform wird nach Standardmethoden (siehe oben) in das entsprechende Acylazid überführt und an die $NH_2$-Gruppe des Trägers in bekannter Weise (siehe oben) gekoppelt. Die überschüssigen Aminogruppen werden dann durch Behandeln mit 5%igem Essigsäureanhydrid in wäßrigem gesättigtem Natriumbicarbonat 20 Minuten lang bei Raumtemperatur acetyliert. Die Kügelchen werden abschließend mit Wasser gewaschen und an der Luft getrocknet.

Beispiel 13:

Man aminiert nach Standardbedingungen ein Carboxylgruppen enthaltendes Polyacrylharz mit einem Gemisch aus Ethylendiamin (21 g) und 1-Cyclohexyl-3-(2-morpholino-ethyl)-carbodiimid-metho-p-toluolsulfonat (15 g) in 100 ml Wasser 24 Stunden lang bei pH 5. Das gewaschene und getrocknete Harz wird nun umgesetzt mit dem Oligosaccharid der Formel (H), an dem, wie oben beschrieben, 8-Hydroxycarbonyloctyl als Linker angekoppelt wurde. Die nicht umgesetzten Aminogruppen des Trägers werden wieder acetyliert.

Beispiel 14:

Humane Leucocyten werden aus heparinisiertem peripherem menschlichen Blut aufgereinigt durch Dextran-Sedimentierung und Ficoll-Hypaque Gradientenzentrifugation. Die aufgereinigten Leucocyten werden in einem für diese Zwecke geeigneten Kulturmedium (z.B. RPMI 1640 Fa. GIBCO, USA) mit einem Zusatz von 5 % humanem Serum resuspendiert (2 x $10^6$ Zellen/ml). Man gibt das Neoglycoprotein gemäß Beispiel 9 hinzu und inkubiert bei 37 °C 4 Tage lang.

Danach gibt man 0.5 µCi $^3H$-Thymidin hinzu und inkubiert weitere 24 Stunden. Die Zellen werden dann geerntet und der radioaktive Einbau an einem Flüssigkeitsszintillationszähler gemessen. Man errechnet eine 58%ige Steigerung der Leucocyten-Proliferation.

Beispiel 15 :

Das Neoglycokonjugat gemäß Beispiel 8 wird in phosphatgepufferter Kochsalzlösung (PBS) gelöst (1 mg/ml) und 1 ml hiervon wird mit 1 ml des vollständigen Freund'schen Adjuvanz vermischt.

Vier Kaninchen werden immunisiert durch Injektion von jeweils 2 x 0.1 ml der Emulsion. Die Injektionen werden dreimal in Intervallen von einer Woche und nochmals nach 3 Monaten wiederholt. Das Serum der immunisierten Tiere wird nach dem ELISA nach polyklonalen Antikörpern untersucht.

Beispiel 16:

Die nach Beispiel 10 erhaltenen Neoglycoproteine werden in phosphat-gepufferter Kochsalzlösung gelöst (1 mg/ml). 1 ml dieser Lösung wird zusammen mit 1 ml complete Freund's adjuvant emulgiert. 10 Wochen alte Weibliche Mäuse werden intraperitoneal mit jeweils 25 µg Antigen immunisiert. Es erfolgen für die Dauer von fünf Wochen Injektionen einmal wöchentlich. Anschließend erfolgen zwei weitere Injektionen im monatlichen Abstand. Die Fusion erfolgt nach der Standardmethode (Kohler und Milstein (1975), Nature 256, 495) drei Tage nach der letzten Auffrischungsinjektion: Die Tiere werden getötet und die Milz entfernt. Isolierte Milzzellen werden mit aminopterin-sensitiven Myelomazellen P3x63Ag8 der Maus im Verhältnis 10:1 in Polyethylenglycol-Lösung (35 %) fusioniert. Hybridoma-Zellen werden in einem Medium kultiviert, das 45 % Hybridoma Medium (GIBCO), 10 % fetales Kälberserum, 45 % Kulturmedium RPMI, sowie Aminopterin enthält. Die einzelnen Zellinien wurden auf ihre Reaktivität gegen das Antigen mittels ELISA (Beispiele 17 und 18) und Immunofärbetechnik untersucht (Beispiel 19).

Beispiel 17

Vorbereitung

Die nach Beispiel 11 hergestellten Neoglycolipide werden in Ethanol gelöst (1 mg/ml), kurz beschallt und anschließend verdünnt mit phosphat-gepufferter Saline pH 7,5 auf eine Endkonzentration von 5 µg/ml.

50 µl dieser Lösung/well werden in eine Polystyrol-Platte (z.B. Fa. Nunc, hochbindend) pipettiert, inkubiert bei 4 °C über Nacht und 3 x mit phosphat-gepufferter Saline + 1 % BSA pH 7,5 gewaschen.

Der Blockierschritt erfolgt durch Zugabe von phosphat-gepufferter Saline + 1 % BSA pH 7,5 1 h, 37 °C.

Durchführung

Jeweils 50 µl Hybridomaüberstand pro well werden pipettiert und 2 h bei 37 °C inkubiert. Ungebundenes Antikörpermaterial wird durch dreimaliges Waschen entfernt.

Der gebundene erste Antikörper wird durch den zweiten mit Peroxidase markierte Antikörper Goat-anti-Mouse-IgG-kappalight-chain-HRPO (1:500 verdünnt) 50 µl/well detektiert. Die Inkubationsdauer beträgt 2 h, 37 °C. Ungebundener Antikörper wird ausgewaschen, wie bereits beschrieben.

Als Nachweisreagenz für Peroxidase dient 0,04 % o-Phenylendiamin in Phosphat/Citrat-Puffer pH 5,0 mit 0,01 % $H_2O_2$, die Messung erfolgt in einem Reader der Fa. SLT bei 492 nm.

Beispiel 18:

ELISA (Enzyme – Linked – Immunosorbent – Assay)

Vorbereitung

Die nach Beispiel 10 hergestellten Neoglycoproteine werden in phosphat – gepufferter Saline pH 7,5 gelöst (5 µg/ml).

50 µl dieser Lösung/well werden in eine Polystyrol – Platte (z.B. Fa. Nunc, hochbindend) pipettiert, inkinbiert bei 4 °C über Nacht und 3 x mit phosphat – gepufferter Saline + 1 % BSA pH 7,5 gewaschen.

Der Blockierschritt erfolgt durch Zugabe von phosphat – gepufferter Saline + 1 % BSA pH 7,5 1 h, 37 °C

Durchführung

Jeweils 50 µl Hybridomaüberstand pro well werden pipettiert und 2 h bei 37 °C inkubiert. Ungebundenes Antikörpermaterial wird durch dreimaliges Waschen entfernt.

Der gebundene erste Antikörper wird durch den zweiten mit Peroxidase markierte Antikörper Goat – anti – Mouse – IgG – kappalight – chain – HRPO (1:500 verdünnt) 50 µl/well detektiert. Die Inkubationsdauer beträgt 2 h, 37 °C. Ungebundener Antikörper wird ausgewaschen, wie bereits beschrieben.

Als Nachweisreagenz für Peroxidase dient 0,04 % o – Phenylendiamin in Phosphat/Citrat – Puffer pH 5,0 mit 0,01 % $H_2O_2$, die Messung erfolgt in einem Reader der Fa. SLT bei 492 nm.

Beispiel 19:

Dünnschichtchromatographie – Immunostaining (TLC – Immunostaining)

Der TLC – Immunostaining – Arbeitsvorgang wird im wesentlichen, wie in der Literatur von Magnani (J.Biol.Chem., 257, 14365, 1982) beschrieben, durchgeführt. Die notwendigen Einzelheiten sind dieser Veröffentlichung zu entnehmen. Neoglycolipide werden durch TLC unter Verwendung von Chloroform/Methanol/0,02 % $CaCl_2$ (60:35:8 oder 55:45:10) analysiert. High performance thin – layer chromatography – Platten, beschichtet mit 0,2 mm Kieselgel 60 (Merck Art. 5548), werden verwendet. Die nach Beispiel 11 hergestellten Neoglycolipide werden in Ethanol 1 mg/ml gelöst und 3 – 5 µl Probenlösung aufgetragen.

Die entwickelten Platten werden luftgetrocknet und mit 2 % – Plexigum – Lösung (Roth, Deutschland) beschichtet. Um unspezifische Bindungen zu vermeiden, wird mit phosphat – gepufferter Saline + 1 % BSA pH 7,5 nachbeschichtet und anschießend wiederum luftgetrocknet.

Die Platten werden mit Kulturüberstand aus Beispiel 16 überschichtet und über Nacht bei Raumtemperatur inkubiert. Ungebundener Antikörper wird ausgewaschen (5 x mit phosphatgepufferter Saline + 1 % BSA pH 7,5). Als zweiter mit Peroxidase markierter Antikörper wird anti – Mouse – IgG (1:500) verwendet. Es wird 2 h bei Raumtemperatur unter sanftem Schütteln inkubiert. Die Platten werden wie oben gewaschen und mit einem Nachweisreagenz (4 – Chlor – 1 – Naphthol in Ethanol und tris – gepufferter Saline mit 0,01 % $H_2O_2$) versetzt. Es wird 15 min unter sanftem Schütteln im Dunkeln inkubiert. Anschießend wird das Nachweisreagenz abgegossen und tris – gepufferte Saline zugefügt. Die visuelle Auswertung zeigt violett – gefärbte Banden, wo sich Neoglycolipide befinden, die mit dem Antikörper reagieren.

**Patentansprüche**

**1.** Antigene Determinanten enthaltende Oligosaccharide im wesentlichen in reiner Form der folgenden Formeln:

(A)

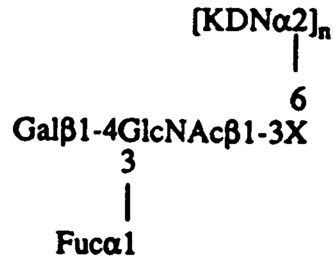

(B)

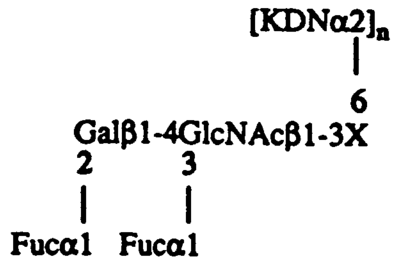

(C)

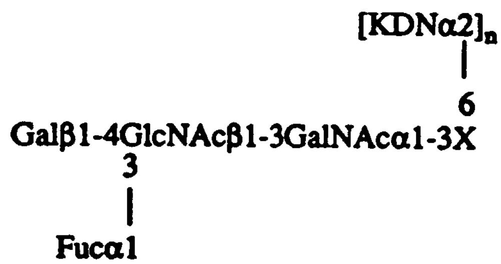

(D)

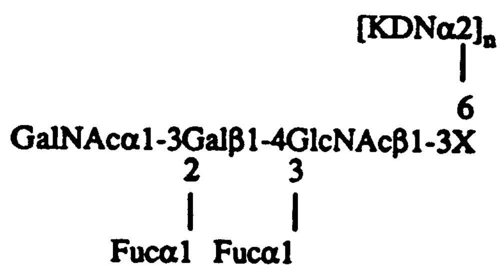

(E)

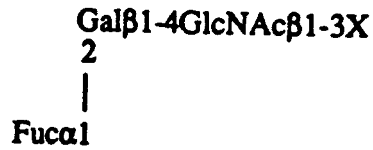

(F)

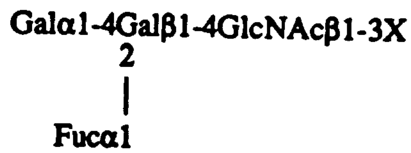

(G)

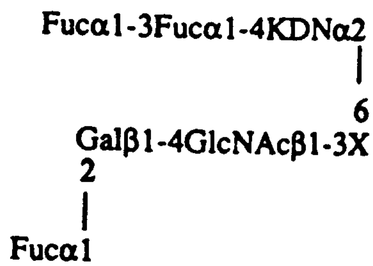

(H)

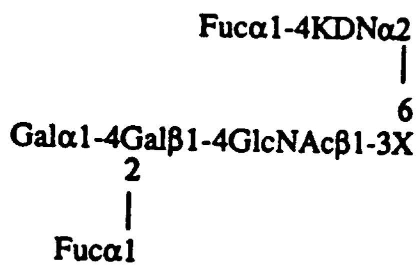

(I)

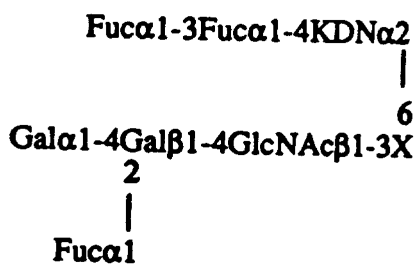

worin

| | |
|---|---|
| X | GalNAc oder GalNAc – ol, |
| n | 0 oder 1, |
| Gal | D – Galactose, |
| GlcNAc | N – Acetyl – D – glucosamin, |
| GalNAc | N – Acetyl – D – galactosamin, |
| GalNAc – ol | N – Acetyl – D – galactosaminitol, |
| Fuc | L – Fucose und |
| KDN | 3 – Deoxy – D – glycero – D – galacto – nonulosonsäure |

bedeuten.

2. Oligosaccharide nach Anspruch 1, dadurch gekennzeichnet, daß in den Formeln (A) bis (D) n = 1 bedeutet.

3. Verfahren zur Herstellung der Oligosaccharide gemäß Anspruch 1, dadurch gekennzeichnet, daß man die Schleimhülle von Eigelegen von Amphibien isoliert und die in ihr enthaltenen Glycoproteine (Mucine) chemisch spaltet und das Gemisch der entstandenen freien Oligosaccharide in nativer Form bzw. in Form der Alditole chromatographisch aufreinigt und gegebenenfalls im Falle der Verbindungen der Formeln (A) bis (D), worin n = 0 bedeutet, den KDN-Rest chemisch abspaltet.

4. Verfahren nach Anspruch 3, dadurch gekennzeichnet, daß die Spaltung der Mucine chemisch mit Hilfe der reduktiven $\beta$-Eliminierung durchgeführt wird.

5. Verfahren nach Anspruch 3 oder 4, dadurch gekennzeichnet, daß als Quelle der Mucine Eigelege von Vertretern aus der Familie der Molche ausgewählt wurden.

6. Verfahren nach Anspruch 5, dadurch gekennzeichnet, daß Vertreter der Gattungen Pleurodeles oder Axolotl, insbesondere die Arten Pleurodeles waltlii oder Axolotl mexicanum ausgewählt werden.

7. Neoglycokonjugat, enthaltend eine Zucker-Komponente und einen Träger, wobei gegebenfalls die Zucker-Komponente über einen Linker an den Träger gekoppelt ist, dadurch gekennzeichnet, daß die Zucker-Komponente ein Oligosaccharid gemäß Anspruch 1 ist.

8. Neoglycoprotein, Neoglycopeptid oder Neoglycolipid nach Anspruch 7.

9. Impfstoff, enthaltend im wesentlichen ein Neoglycokonjugat gemäß Anspruch 7 oder 8.

10. Mono- oder polyklonaler Antikörper, dadurch gekennzeichnet, daß er spezifisch auf eine antigene Determinante eines Oligosaccharides gemäß Anspruch 1 gerichtet ist.

11. Tumorassoziertes Antigen, dadurch gekennzeichnet, daß es ein Epitop enthält, an das ein mono- oder polyklonaler Antikörper gemäß Anspruch 10 bindet.

12. Immunoadsorbens zur Aufreinigung von Antikörpern gemäß Anspruch 10, bestehend aus einer Trägermatrix, an die ein Oligosaccharid der Formeln (A) bis (I) gekoppelt ist.

13. Immunoadsorbens zur Aufreinigung von Oligosacchariden der Formeln (A) bis (I), bestehend aus einer Trägermatrix an die ein Antikörper gemäß Anspruch 10 angekoppelt ist.

14. Verfahren zur in vitro-Bestimmung von Immunogenität durch antigen-spezifische Stimulation von isolierten Lymphocyten, dadurch gekennzeichnet, daß man die Lymphocyten mit dem Mucin aus der Schleimhülle von Amphibien-Eiern oder mit einem Neoglycokonjugat gemäß Anspruch 7 oder 8 rekultiviert und die Proliferation der Lymphocyten durch Aufnahme von $[^3H]$-Thymidin mißt.

15. Verwendung der Schleimhülle von Eigelegen von Amphibien zur Gewinnung von Oligosacchariden mit dem Strukturelement Gal($\beta$1-4) - GlcNAc($\beta$1-3), wobei mindestens zusätzlich ein Fuc-Rest und mindestens ein weiterer Gal-, GalNAc-, GlcNAc-, Fuc- oder KDN-Rest mit den oben angegebenen Bedeutungen vorliegt.

16. Verwendung der Schleimhülle von Eigelegen von Amphibien zur Herstellung von anti-$Le^X$-, anti-$Le^Y$- und anti-KDN-Antikörpern.

17. Verwendung der Schleimhülle von Eigelegen von Amphibien zur Herstellung von 3-Deoxy-D-glycero-D-galacto-nonulosonsäure (KDN).

18. Verwendung der Schleimhülle von Eigelegen von Amphibien zur Bereitstellung eines Immunoassays.

19. Verwendung der Schleimhülle von Eigelegen von Amphibien zur Herstellung eines Impfstoffes für die Behandlung von Tumoren.

**20.** Verwendung nach einem der Ansprüche 15 – 19, dadurch gekennzeichnet, daß man die Schleimhülle von Eigelegen von Amphibien der Familie der Molche, insbesondere der Gattungen Pleurodeles und Axolotl einsetzt.

Fig. 1

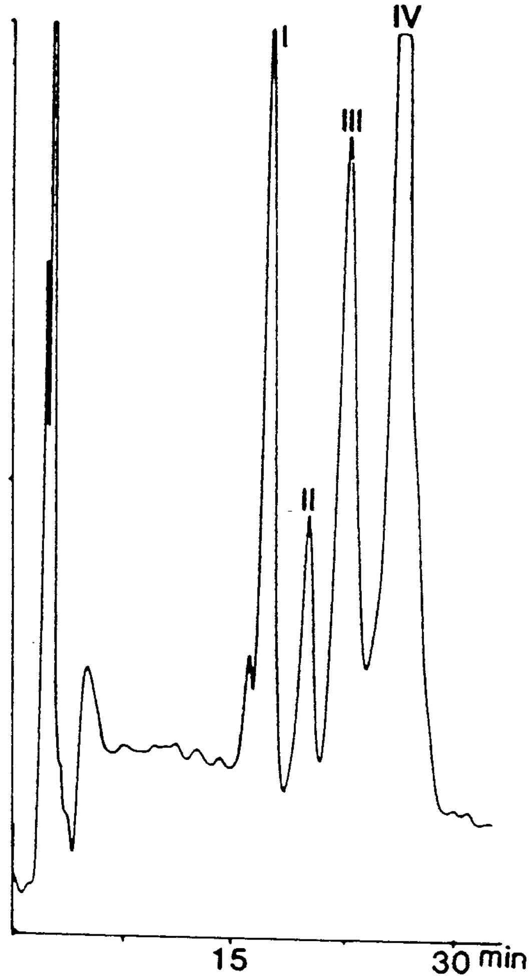

Fig. 2

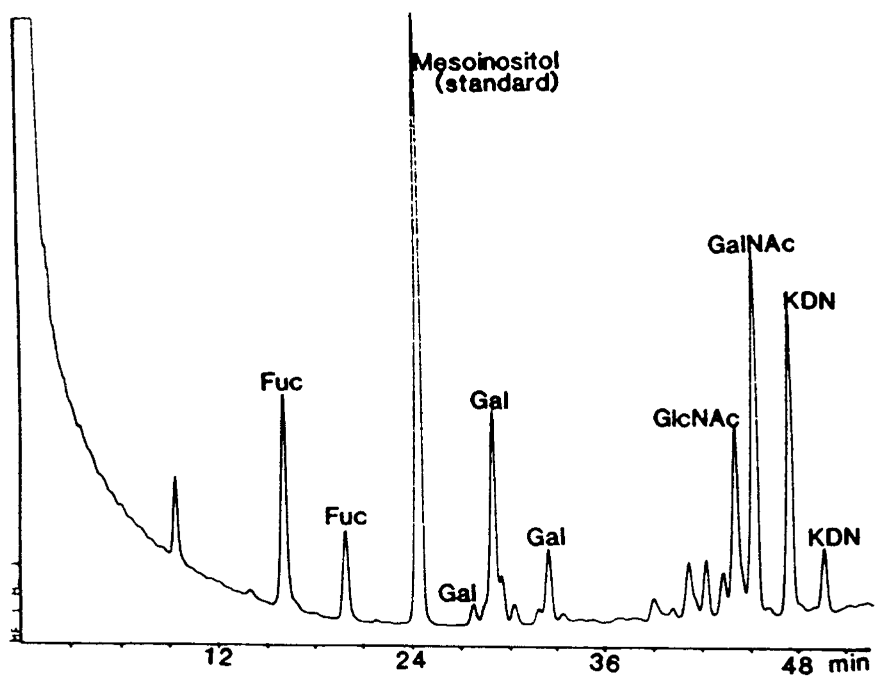
Mesoinositol
(standard)
GalNAc
KDN
Fuc
Gal
GlcNAc
Fuc
Gal
KDN
Gal
12
24
36
48 min

Fig. 3

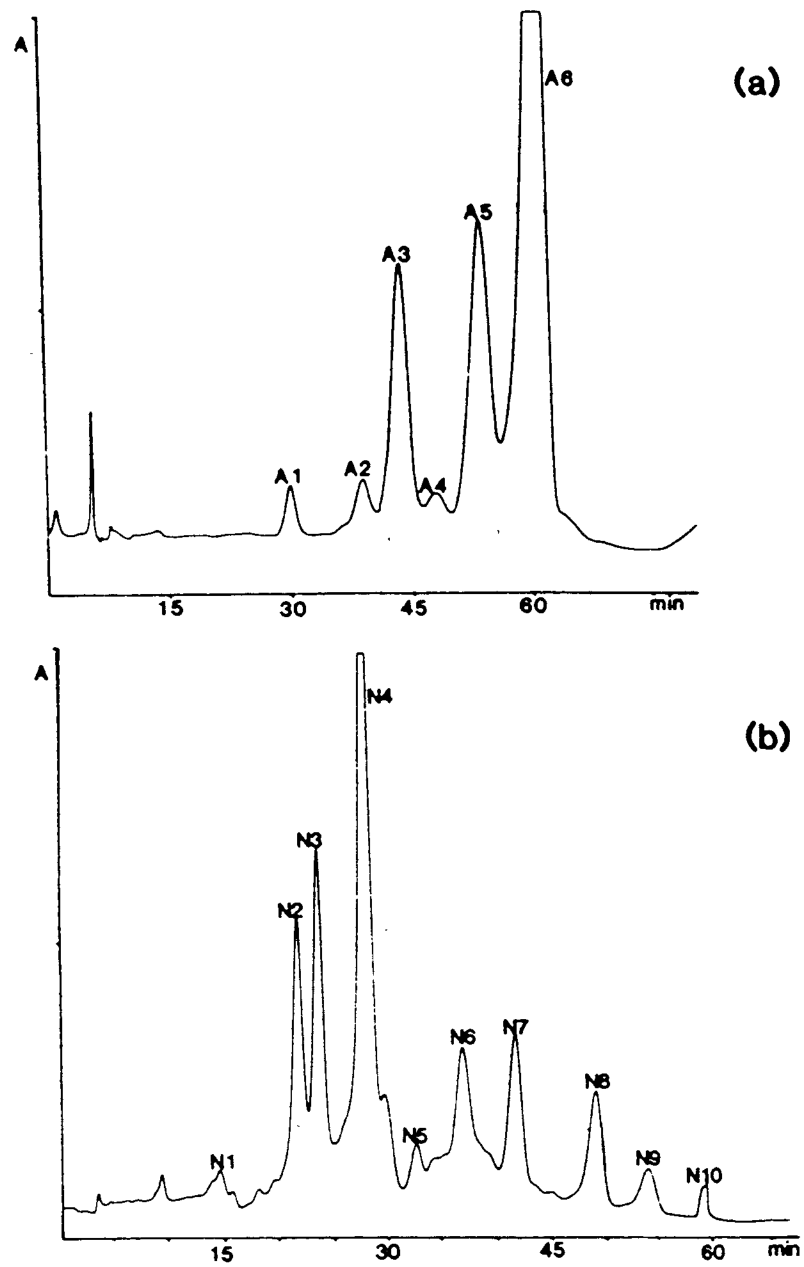
A
(a)
A1
A2
A3
A4
A5
A6
15
30
45
60
min
A
(b)
N1
N2
N3
N4
N5
N6
N7
N8
N9
N10
15
30
45
60
min

Fig. 4

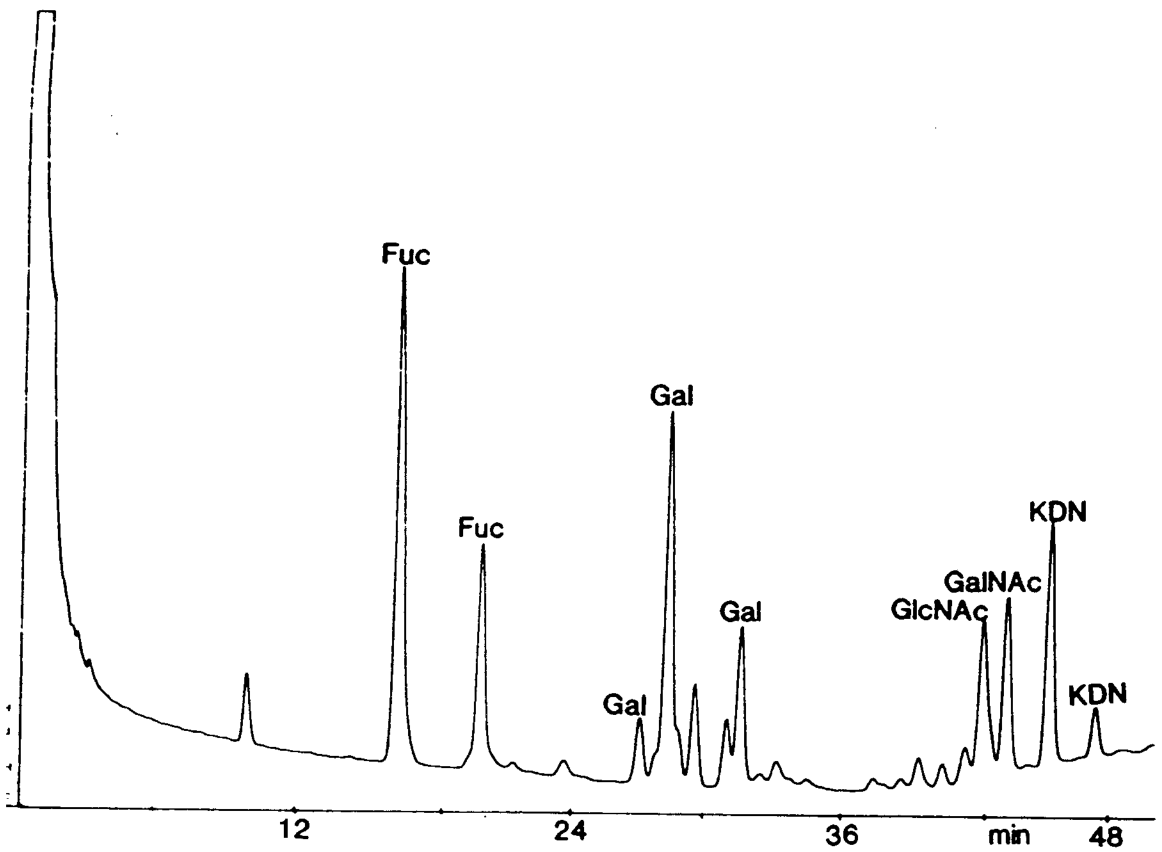

Europäisches Patentamt

# EUROPÄISCHER RECHERCHENBERICHT

Nummer der Anmeldung

EP 92119025.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.5) |
|---|---|---|---|
| P,X | CHEMICAL ABSTRACTS, Band 116, Nr. 15, 13. April 1992 Columbus, Ohio, USA GERARD STRECKER et al. "Characterization of Le x,Le y and A Le y antigen determinants in KDN-containing O-linked glycan chains from Pleurodeles waltlii jelly coat eggs", Seite 678, Spalte 1, Zusammenfassung-Nr. 149 664r & FEBS Lett. 1992, 298(1), 39-43 | 1-6, 11,15, 17,20 | C 07 H 5/06<br>C 07 H 1/08<br>C 07 K 15/14<br>C 07 K 15/16<br>C 07 K 15/28<br>A 61 K 39/385<br>G 01 N 33/541 |
| A | CHEMICAL ABSTRACTS, Band 73, Nr. 25, 21. Dezember 1970 Columbus, Ohio, USA J. PEREDA "Histochemical study of the distribution of sialomucins in the oviduct and jelly coat of the oocytes of Rana pipiens. Behaviour of the different layers of the coat in water.", Seite 129, Spalte 1, Zusammenfassung-Nr. 128 199w & J. Embryol. Exp. Morphol. 1970, 24(Pt.1), 1-12(Fr) | 1,2 | RECHERCHIERTE SACHGEBIETE (Int Cl.5)<br>C 07 H 1/00<br>C 07 H 5/00<br>C 07 K 15/00<br>A 61 K 39/00<br>G 01 N 33/00 |
| A | CHEMICAL ABSTRACTS, Band 72, Nr. 21, 25. Mai 1970 Columbus, Ohio, USA J. PEREDA "Histochemical study of the mucopolysaccharides of the oviduct and of the jelly coat of the ovocytes of Rana pipiens: incorporation of 35S-labeled | 1,2 | |

Der vorliegende Recherchenbericht wurde für alle Patentansprüche erstellt.

| Recherchenort | Abschlußdatum der Recherche | Prüfer |
|---|---|---|
| WIEN | 23-02-1993 | SCHARF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER RECHERCHENBERICHT

Nummer der Anmeldung

-2-
EP 92119025.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| | sulfate",<br>Seite 124, Spalte 2,<br>Zusammenfassung-Nr. 108 323h<br>& Develop. Biol. 1970,<br>21(3), 318-30(Fr.)<br>---- | | |

RECHERCHIERTE SACHGEBIETE (Int. Cl.⁵)

Der vorliegende Recherchenbericht wurde für alle Patentansprüche erstellt.

| Recherchenort | Abschlußdatum der Recherche | Prüfer |
|---|---|---|
| WIEN | 23-02-1993 | SCHARF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument